# EUROPEAN PATENT APPLICATION

(11) **EP 4 782 814 A1**
(43) Date of publication of application: **29.07.2026**
(21) Application number: 25153931.8
(22) Date of filing: 24.01.2025
(51) Int. Cl.: G01N 15/149, C12M 1/00, B01L 9/00, C12M 1/36, G01N 35/10

(54) **SORTING APPARATUS AND CORRESPONDING SORTING METHOD**

(71) Applicant: CSEM Centre Suisse d'Electronique et de Microtechnique SA - Recherche et Développement, 2002 Neuchâtel (CH)
(72) Inventor: GOLDOWSKY, Jonas, 6030 Ebikon (CH); JANDET, Lucie, 6003 Luzern (CH); SHYNKARENKO, Yevhen, 7302 Landquart (CH)
(74) Representative: e-Patent SA

(57) **Abstract**

A biological entities sorting apparatus (1) is disclosed, which comprises a picking head (14) carrying a picking tip (16) defining a picking axis, and an illumination device (30) arranged to cooperate with a camera (26) via a given optical path (32). The illumination device (30) and the camera (26) can be driven to move to an appropriate position for carrying out the acquisition of images of a biological entity to pick up from a picking position in a sample, while the picking head (14) can be driven to move and place the picking tip (16) it carries in a picking configuration, in which it may pick up the biological entity of interest while being kept out of the corresponding optical path (32) so that images can be acquired during the picking procedure.

## Description

### Technical Field

The invention is in the field of the handling and sorting of sensitive biological entities, essentially multicellular entities, such as cell aggregates, cell colonies, organoids, spheroids, zebrafish eggs, zebrafish larvae, c. elegans, tissue sections, biopsy tissue, assembloids or others, herein generally named by "biological entities" in the context of the present disclosure.

More specifically, the invention relates to a sorting apparatus, adapted to allow selective picking up of at least one biological entity from a vessel containing a sample including a plurality of biological entities, the sorting apparatus comprising:
- a picking station including a frame, on which a vessel support having a see-through window is mounted for bearing at least one vessel, as well as a picking head having a connecting member for securing an elongated picking tip intended to define a picking axis, the picking head being mounted on the frame in such a manner that it can be driven to move with respect to the frame to be able to place the elongated picking tip in a picking configuration, in which it can pick up a predefined biological entity from a picking position inside a vessel while the latter is located on the vessel support,
- an image acquisition device arranged so as to be able to acquire through the see-through window an image of at least part of biological entities included in a sample contained in a transparent vessel while the latter is located on the vessel support, and
- an illumination device arranged so as to be able to illuminate at least partly a sample contained in a vessel while the latter is located on the vessel support.

### State of the art

Among the multicellular entities of the kinds previously mentioned, organoids are three-dimensional structures grown from stem cells that typically mimic the structure and function of organs. They are generally cultivated in laboratory settings under controlled conditions, allowing researchers to study aspects of organ development, disease progression, and drug responses in a more accurate and ethically acceptable manner compared to traditional animal models or cell cultures. Those biological entities represent a powerful tool in biomedical research with diverse applications spanning from basic science to clinical translation. The size of such a biological entity can range from very small structures resembling a few hundred micrometers in diameter to larger, more complex structures that can be several millimeters in size.

Depending on factors like cell source, culture conditions, culture techniques among others, organoids with very heterogeneous properties can result from their maturation in the same culture sample. Many applications, e.g. drug screening, require a selection step, including quality control and individualization (i.e. separation) of the selected biological entities before the biological assays can be run. The need for separation and sorting is also encountered in the culture and handling of other small biological entities including organisms such as zebrafish eggs, larvae or c. elegans, which are also commonly used in biomedical research.

There are several commercial solutions for the inspection and picking of individual cells, small-sized multicellular entities and even organoids. See for example, the "Cellcelector flex" by Sartorius: https://www.sartorius.com/en/products/cell-selection-and-retrieval?_gl=1*28qq1t*_up*MQ..&gclid=Cj0KCQjw0MexBhD3ARlsAEI3WHL Zg3NpyAWDNuTQyccNMEdAHXBzw0Th0IV5y6FTW8teK-igBTHTv9YaAgCDEALw_wcB.

Patent EP2955502B1 discloses a similar automated sorting apparatus including the above-mentioned features. More particularly, this sorting device comprises an image acquiring system arranged to identify and locate biological entities of interest in a sample, this system including here an annular backlight illumination device intended to cover the full extent of the source vessel containing the sample. Once a mapping of the sample is done, the source vessel is moved with high precision, along the xy plane, to bring a biological entity to be selectively picked up in a picking or suction position, where it can be picked up without interference between the picking head and the illumination device.

In general, the handling of such biological entities with sizes larger than 1 mm (up to 5 mm) is difficult because of their tendency to sediment and their fragility to mechanical stress. Consequently, the movements of the source vessel have to be carried out very carefully in such an apparatus, which leads to necessary compromises between the corresponding time consumption and the risk of damages to the biological entities contained in the sample. Accordingly, such sorting devices are typically arranged so as to be able to cautiously move a source vessel from an identification station to a picking station, for instance, after a localization map of the biological entities of interest in a given sample is built up, these devices are generally adapted to move the source vessel by implementation of high resolution linear x, y actuators, in a smooth and slow manner so as to avoid as much as possible any displacement of the biological entities within the sample.

US Patent 8,947,518 B2 discloses another sorting apparatus for similar biological entities, with a more sophisticated imaging system. Indeed, this imaging system comprises a macro-image capturing system, arranged above a source vessel support, as well as a micro-image capturing system arranged below the source vessel support. An oblique illumination device is also provided on the frame of the apparatus, next to the macro-image capturing system. In a similar way to that of the first mentioned apparatus, the support for the source vessels is arranged to be movable in the xy plane with respect to the frame of the apparatus. A picking unit is further arranged on the frame, next to a dispensing station where picked up entities may be delivered then. Though the picking unit comprises a picking head arranged so that it can move along the z direction, on top of being able to turn around the same z axis, this apparatus is built to have a specific and unique picking axis corresponding to the common imaging axis of the micro-image and of the macro-image capturing systems. Hence, once a biological entity to pick up has been identified, the support of the source vessel needs to be moved along the xy plane to place the biological entity of interest on the imaging axis, which raises the same problems as with the previously described apparatus, i.e. risks of misalignment and of damages of the biological entities in the sample.

Patent publication WO2023/233464 A1 discloses several embodiments of a sorting apparatus for biological entities having a different construction approach than that of the preceding apparatuses. According to a first embodiment illustrated in Figure 1 of WO2023/233464 A1, this apparatus may comprise an imaging system arranged below a transparent plate defining a support for source vessels and for destination vessels, in such a manner that it can be driven between a source vessel and a destination vessel. A picking unit is arranged above the transparent plate in such a manner that its picking head can be placed above a source vessel, to pick up at least one biological entity, before being driven above a destination vessel to dispense the picked up biological entity therein. Thus, the imaging system may first acquire images of the picking up operation, while it is positioned below the source vessel, before being driven below the destination vessel for carrying out the acquisition of images during the dispensing operation. Thanks to this specific approach, this apparatus avoids the risk of damages to the biological entities contained in a sample of interest.

However, the first embodiment lacks an illumination device which is generally necessary to ensure a sufficient quality of the imaging results, or to reveal specific features of certain biological entities for the purpose of identifying them, for instance by fluorescence illumination. Another embodiment of this apparatus, as illustrated in Figure 14 of the above-mentioned publication, provides that the imaging system further comprises an illumination device coupled with the imaging device, in such a way that both devices are facing each other with either a source vessel or a destination vessel interposed between them, on their optical path. The illumination device and the imaging device are arranged to be movable along the axis extending between the position of the source vessel and the position of the destination vessel. Likewise, a picking unit is arranged so as to be movable in the xy plane to be able to move between the source vessel and the destination vessel, as well as along the z axis to place picking tips in corresponding picking or dispensing configurations. However, it appears quite clearly from the disclosure of this publication that the picking unit and the illumination device might interfere with each other in the case of such a construction, which implies that they have to function according to a sequential basis, i.e. according to the following sequence:
- an image of the source vessel is acquired,
- the imaging system is moved away from the source vessel,
- the picking unit is driven above the source vessel,
- the biological entity of interest is selectively picked up,
- the picking unit is moved away from the source vessel,
- the imaging system might be aligned again with the source vessel to acquire a new image, for the purpose of checking that the biological entity of interest was correctly picked up,
- the picking unit is driven above the destination vessel,
- the biological entity of interest is delivered in the destination vessel,
- the picking unit is moved away from the destination vessel, and
- the imaging system is aligned again with the destination vessel to acquire an image thereof, for the purpose of checking that the biological entity of interest was correctly delivered.

Even more steps are necessary if the biological entity of interest is not correctly picked up or delivered. However, though it requires many different steps and moves, this approach might still be interesting in comparison to those of the previously described apparatuses, as far as the displacements of the picking unit and of the components of the imaging system can be much faster than those of the vessels carrying the sample or the delivered biological entities. Thus, the full operation might require less time, all in all, than with the previously described apparatuses.

It appears from what precedes that the need still exists to provide a solution to be able to selectively pick up biological entities in a fast and reliable manner from a sample, without taking risks of damaging any of the biological entities included in this sample.

### Disclosure of the invention

An aim of the present invention is to propose a sorting apparatus making it possible to selectively pick up biological entities from a sample with reduced risks of damages that could be caused to one or several of the biological entities included in this sample. Another aim of the present invention is to propose a method taking advantage of the above apparatus for the selective picking up of at least one biological entity from a source vessel containing a sample including a plurality of biological entities, for instance to be able to selectively dispense this picked up biological entity into a destination vessel, in a precise, reliable and non-damaging manner.

These aims are achieved by providing a sorting apparatus as mentioned above, further characterized
by the fact that the image acquisition device and the illumination device are mounted on the frame in such a manner that they can move with respect to the frame to define between them different optical paths intersecting the see-through window and defining corresponding imaging axis, and
by the fact that the picking head, the illumination device and the image acquisition device are arranged so that, for any picking position in a vessel, which faces the see-through window while the vessel is located on the vessel support, an elongated picking tip secured to the picking head can be placed in an appropriate picking configuration with its picking axis intersecting the imaging axis substantially at the picking position, such that the elongated picking tip can be kept out of the corresponding optical path between the illumination device and the image acquisition device during a picking up operation.

It thus appears that according to a different approach compared to prior devices, the sorting apparatus of the present invention allows the implementation of a real time imaging of great quality of the source vessel, more particularly of a biological entity to be picked up included in a sample contained in the source vessel. Indeed, thanks to the construction of the apparatus according to the present invention, the image acquisition device, the illumination device and the picking head are arranged so that the picking head can be driven to carry out the picking operation without interfering with the optical path between the illumination device and the imaging device during the picking operation. Thanks to these features, not only the integrity of the biological entities included in the sample is safeguarded and the consistency of the sample locations is assured, but also the whole sorting operation requires a minimum of back-and-forth movements of the moving components of the apparatus, which allows savings of time and energy.

According to a preferred embodiment of the invention, the picking head and the illumination device may preferably be carried by a supporting arm mounted on the frame in such a manner that it can be driven in translation inside a xy plane with respect to the frame, i.e. in a first plane substantially parallel to the vessel support.

In this case, the image acquisition device may also preferably be driven in translation with respect to the frame inside a second plane substantially parallel to the vessel support.

Advantageously, it may further be provided that the image acquisition device is mounted on the frame via the supporting arm, so that its movements inside the second plane are synchronized with the movements of the illumination device in the first plane.

Generally, the illumination device and the image acquisition device may preferably be connected to the frame in such a manner that each of them can be tilted with respect to the frame.

Generally, the picking head and the illumination device may advantageously be arranged so that the picking axis and the imaging axis can intersect at the picking position with an angle α comprised between 5° and 140°, based on geometrical and optical reasons, as will be explained later in the detailed description.

Generally, the image acquisition device may preferably include a reflective member to change the light propagation direction with respect to the imaging axis.

In a similar way, the image acquisition device may preferably include an adjustable focusing system adapted to take into account different possible distances between entities to be picked up and the vessel support.

According to a preferred embodiment, the imaging axis may have a same angle with respect to the see-through window for different picking positions. More specifically, the imaging axis may be inclined, in this case, with a same inclination angle with respect to the normal direction to the see-through window for different picking positions. The picking tip might then simply be driven along the z axis (substantially perpendicular to the vessel support) in this case to be placed in and out of the picking position.

On a general basis, the image acquisition device may preferably be adapted for microscopy imaging, preferably for bright field imaging and/or fluorescence imaging.

According to a preferred embodiment, the sorting apparatus of the present invention may further be arranged in such a manner
that it comprises a delivery station including a destination vessel support having a see-through window for bearing at least one destination vessel,
that the picking head can be driven in such a manner that an elongated picking tip secured thereon, intended to define a delivery axis, can be placed in a delivery configuration in which a picked-up predefined biological entity can be delivered in a predefined delivery position inside the destination vessel,
that the illumination device and the image acquisition device can be driven to define an optical path through the see-through window of the destination vessel support with a predefined imaging axis, and
that the picking head, the illumination device and the image acquisition device are arranged so that, for any delivery position in a destination vessel, which faces the see-through window while the destination vessel is located on the destination vessel support, an elongated picking tip secured to the picking head can be placed in a corresponding delivery configuration with its delivery axis intersecting the imaging axis substantially at the delivery position, such that the elongated picking tip can be kept out of the corresponding optical path between the illumination device and the image acquisition device during a delivering operation.

The present invention further relates to a method for the selective picking up of at least one biological entity from a source vessel containing a sample including a plurality of biological entities.

More specifically, the method of the present invention advantageously comprises the steps consisting in:
a) providing a sample including biological entities in a transparent source vessel and place the latter on a vessel support of a sorting apparatus according to the previously disclosed features,
b) controlling the illumination device and the image acquisition device to move them, while keeping the vessel support immobile, so as to define an optical path including a predefined biological entity to be picked up, defining a picking position, and intersecting the see-through window with a corresponding imaging axis,
c) acquiring at least one image of the predefined biological entity to be picked up, and
d) controlling the picking head, on which an elongated picking tip is secured, so as to place the elongated picking tip in a picking configuration in which it can pick up the predefined biological entity from the vessel, with its picking axis intersecting the imaging axis substantially at the picking position, the elongated picking tip being kept out of the optical path between the illumination device and the image acquisition device,
e) selectively picking up the predefined biological entity, and acquiring at least one image of the predefined biological entity while it is moving between the picking position in the sample and the elongated picking tip.

According to a preferred embodiment, the illumination device and the image acquisition device are advantageously controlled to move in a substantially synchronized manner in step b).

According to another preferred embodiment, the illumination device and the image acquisition device are advantageously controlled to maintain a substantially constant angle between the imaging axis and the see-through window while they are moving in step b).

According to another preferred embodiment, when the sorting apparatus comprises a delivery station according to the above-mentioned features, the method according to the invention may advantageously comprise additional steps consisting in:
f) controlling the illumination device and the image acquisition device to move them so as to define an optical path including a predefined delivery position, and intersecting the see-through window of the destination vessel support with a corresponding imaging axis,
g) controlling the picking head so that it can place the elongated picking tip in a delivery configuration corresponding to the predefined delivery position, the elongated picking tip being kept out of the optical path between the illumination device and the image acquisition device,
h) delivering the predefined biological entity in the delivery position, and acquiring at least one image of the predefined biological entity while it is moving between the elongated picking tip and the delivery position.

### Brief description of the drawings

Further details of the invention and other advantageous embodiments will appear more clearly upon reading the description below, in connection with the following figures which illustrate:
- Fig. 1: Schematic perspective overall view of a sorting apparatus according to a preferred embodiment of the present invention;
- Figs. 2a and 2b: Schematic and simplified lateral view of a sorting apparatus according to a first variant embodiment of the present invention in two respective different configurations;
- Figs. 3a and 3b: Schematic and simplified lateral view of a sorting apparatus according to another variant embodiment of the present invention in two respective different configurations;
- Figs. 4a and 4b: Schematic and simplified lateral view of a sorting apparatus according to another variant embodiment of the present invention in two respective different configurations, and
- Fig. 5: Schematic and simplified lateral view of a sorting apparatus according to another variant embodiment of the present invention.

### Embodiments of the invention

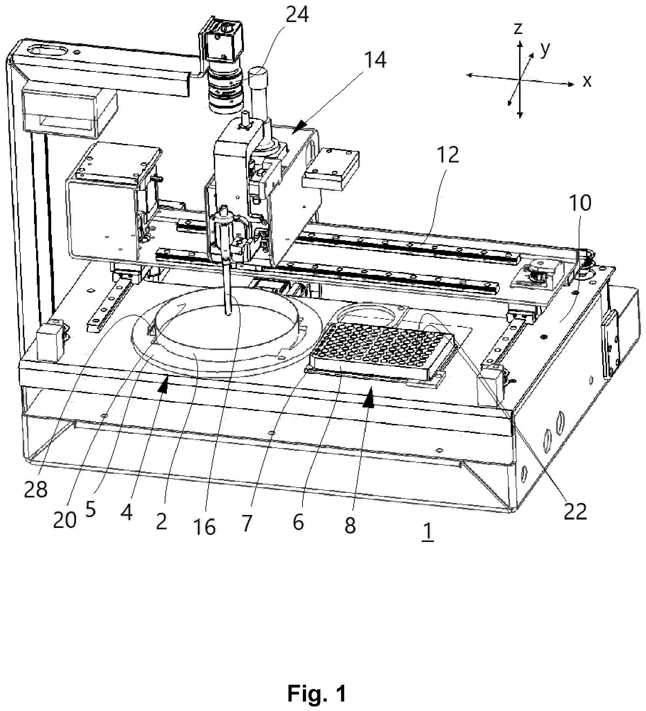

Fig. 1 is a schematic perspective overall view of a sorting apparatus 1, for sorting biological entities contained in a sample, according to a preferred embodiment of the present invention, in which most of the main components of the sorting apparatus 1 are illustrated.

The sorting apparatus 1 is an inspection and sorting apparatus for sorting biological entities, essentially multicellular entities, such as cell aggregates, cell colonies, organoids, spheroids, zebrafish eggs, zebrafish larvae, c. elegans, tissue sections, biopsy tissue, assembloids or others, advantageously by selective picking up of at least one biological entity from a source vessel 2, containing a sample including a plurality of biological entities and located in a picking station 4, to be able to selectively dispense this biological entity into a destination or target vessel 6 located in a delivery station 8.

The sorting apparatus 1 comprises a main housing defining a frame 10 and preferably adapted to be installed on a working surface (not illustrated), for instance a table in a laboratory.

The housing is typically provided with different electronic interfaces, at least for power supply, and contains electronic circuits essentially having a well-known architecture in an advantageous manner.

The frame 10 essentially includes the upper side of the housing which delimits here the picking station 4 and the delivery station 8, adjacent to each other in an illustrative non-limiting way.

In Fig. 1, the source vessel 2 is represented in its functional configuration in the picking station 4, borne by a vessel support 5, while the destination vessel 6 is represented in its functional configuration in the delivery station 8, borne by a destination vessel support 7.

The frame 10 further carries a guiding structure 12 for a picking head, here a pipetting head 14, movable in the xy plane and comprising at least one elongated picking tip or pipette tip 16 movable in the z direction (either alone, with reference to the pipetting head 14, or together with it). In a known manner, the pipetting head 14 can typically be controlled to move the pipette tip 16 between the picking station 4 and the delivery station 8, where the pipette tip 16 is driven to move along the z direction, to pick up one or several biological entities from the source vessel 2 and deliver them in the destination vessel 6. It thus appears that, in an illustrative and non-limiting manner, the picking head 14 is movable here inside a plane substantially parallel to the general plane of the support vessel 5, while the pipette tip 16 is movable along an axis that is substantially perpendicular to the general plane of the vessel support 5.

It should be noted that both source vessel 2 and destination vessel 6 are shown with illustrative and non-limiting shapes. Typically, the source vessel 2 may have one or several large cylindrical wells 20 (only one here in a non-limiting illustrative way), each intended to contain a sample (with a liquid or gel) including several biological entities, while the destination vessel 6 has more smaller wells 22, each of which is intended to contain one picked up biological entity only (sometimes, two or more similar biological entities might be delivered in a same receiving well 22 in a known manner). Of course, the source and destination vessels may have different alternative shapes without going beyond the scope of the invention as defined in the appended claims. For instance, the source vessel may have several wells as mentioned above and/or the delivery station 8 might be provided with a support arranged for supporting a plurality of individual vessels, each defining one receiving well 22. More generally, the sample vessels or plates may comprise petri dishes, 6 well-plates, 96 well plates, or any other standard or custom culture recipient.

The sorting apparatus 1 further comprises an identification and localization system allowing to carry out an assessment of the content of a sample contained in a given source vessel 2 as it is installed in its functional configuration in the picking station 4. Many commercially available systems are already known for mapping the content of a sample, generally not only the number of biological entities included in the sample and their precise location or distribution within the source vessel, but also the composition of each biological entity included in the sample. Each biological entity of interest might define a specific picking position in the sample.

For instance, such an identification and localization system may preferably comprise an overview camera 24 arranged above the picking station 4. It should be noted that the vessel support 5 may advantageously include a dark field illumination used to generate high contrast images of the biological entities located within the source well(s) 20.

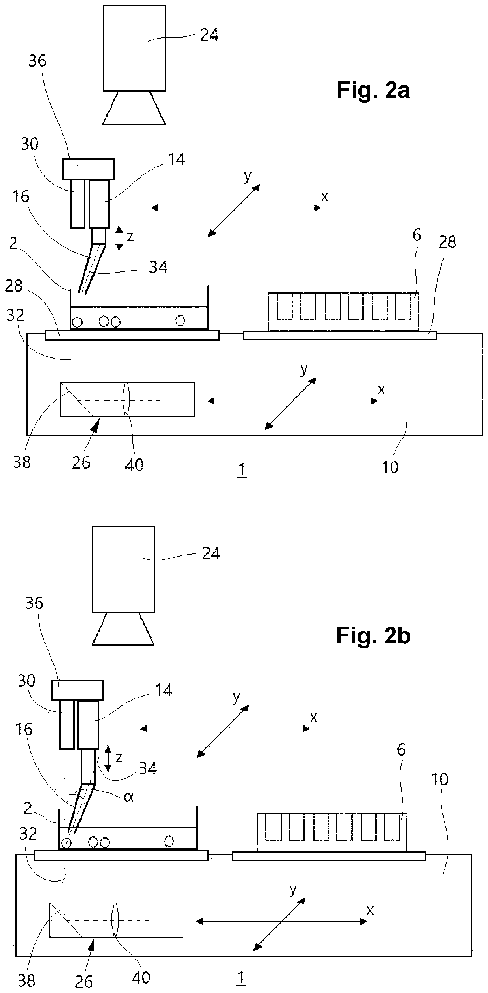

Figs. 2a and 2b are schematic and simplified lateral views of the sorting apparatus 1, from which it appears that, optionally, the identification and localization system might advantageously comprise an additional camera 26 arranged in the opposite direction to that of the overview camera 24.

Either or both of these cameras 24, 26 might be adapted to acquire a magnified image of the content of the sample in order to properly assess the distribution in the source vessel 2 of the plurality of biological entities included in the sample. One camera might be arranged so as to acquire images from one of the picking station 4 and the delivery station 8 while the other camera might be arranged to acquire images from the other station. In fact, it also makes sense to assess the content of the destination vessel 6 after a biological entity was allegedly delivered, to check if it is indeed the case.

According to a preferred embodiment, the two cameras 24, 26 may have different optical properties (in terms of resolution and/or magnification but also in terms of light spectrum) but one of them might be still with respect to the frame 10, while the other one might be movable between the picking station 4 and the delivery station 8. More specifically, according to the present invention, the overview camera 24 might be maintained immobile above the picking station 4 and the additional camera 26 might be mounted on the frame 10 in such a way that it can be moved to scan the picking station 4, as well as preferably to move between the picking station 4 and the delivery station 8, advantageously to follow the pipetting head 14. Thanks to such an arrangement, cameras 24 and 26 might simultaneously provide images of the picking station 4, at the time of picking up a biological entity from a sample, where the corresponding images would provide complementary data, for instance for the purpose of identifying the biological entities included in the sample. Then, the additional camera 26 might follow the pipetting head 14 to the delivery station 8, to be able to check that the delivery of a given biological entity in a destination vessel 6 is properly carried out. Advantageously, the camera 26 may comprise a microscope system.

Generally, the housing of the sorting apparatus 1 may advantageously comprise one or several see-through windows 28 so that the camera 26 may acquire images of the content of the source vessels 2 and of the destination vessels 6 from below, i.e. from the side of the vessels which is opposite to that of the pipetting head 14. For that purpose, different alternative embodiments may be implemented without any major impact on the present invention. For instance, the housing may comprise portions made of a transparent material (glass or plastics) located in the picking station 4 and/or in the delivery station 8, or the window(s) might even be simply implemented in the form of openings (or at least one could be implemented in the form of an opening and the other might be implemented in the form of a portion made of a transparent material). In general, a transparent part for defining each see-through window 28 is preferred to avoid any risk that part of a sample could be spilled on the camera 26.

Thanks to the preceding features, it is possible to take advantage of the fact that most vessels (culture dishes and multi-well plates) are normally transparent, by running the acquisition of images through their bottom. The one skilled in the art will encounter no particular difficulty to adapt the present teaching to his own needs and implement alternative solutions to those which have just been described, as far as the implementation details of the general architecture of the sorting apparatus 1 is concerned, for instance the features relating to its housing, without going beyond the scope of the invention as defined by the appended claims.

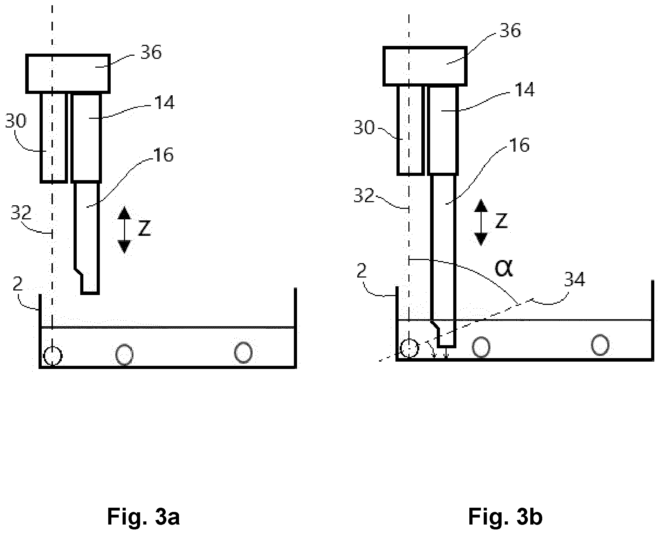

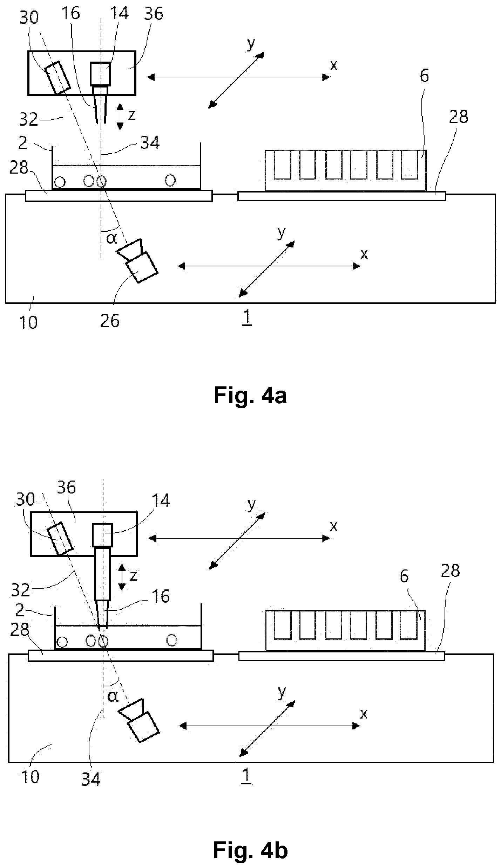

Now referring back to the main goals of the apparatus according to the present invention, the corresponding solution provided by the present invention will be more precisely explained in connection with Figs. 2a and 2b, as well as with Figs. 3a, 3b, 4a and 4b.

As already mentioned earlier, the provision of an illumination device 30, preferably of the backlight type, is required to ensure an optimal result for the identification and localization of the biological entities included in a sample contained in a source vessel 2, more especially when the sorting apparatus 1 is provided with an image acquisition device like the camera 26 adapted to carry out microscopy imaging. Preferably, the illumination device 30 can provide a backlight which may be for instance collimated light implemented with LED and lens(es).

According to the present invention, it is possible to avoid the problems of the prior art as previously presented, if the picking head 14, the illumination device 30 and the image acquisition device, here the camera 26, are arranged in a specific way with respect to each other.

More precisely, the illumination device 30 has to be arranged so as to be able to illuminate at least partly the sample contained in a source vessel 2 when the latter is placed on the vessel support 5, and so as to define different optical paths 32 between the illumination device 30 and the camera 26 intersecting the see-through window 28 and defining corresponding imaging axes. Moreover, the picking head 14, the camera 26 and the illumination device 30 are advantageously arranged so that, for any picking position in the source vessel 2, which faces the see-through window 28, the pipette tip 16 can be placed in a picking configuration, in which it can pick up a predefined biological entity of interest, while the pipette tip 16 defines a picking axis 34 which intersects the imaging axis (or corresponding optical path 32) substantially at the picking position, i.e. at the position of the biological entity to be picked up. In the present disclosure, the picking axis 34 should be interpreted as being defined along the flow of the sample in the area of the opening of the pipette tip 16. The picking axis 34 thus depends on the orientation of the tip opening rather than on the general longitudinal direction of the pipette tip 16, as will be more clearly apparent from the description below.

Thanks to those features, the pipette tip 16 can be kept out of the corresponding optical path 32 between the illumination device 30 and the camera 26, allowing thus the real time acquisition of images before, during and after the picking operation.

The intersection angle α between the imaging axis and the picking axis is preferably comprised approximately between 5° (to ensure that the pipette tip 16 does not impinge the optical path during the picking up of the biological entity of interest) and 140°, to ensure a proper propagation of the light from the illumination device 30 to the camera 26, through the different interfaces to go through. More precisely, a maximum incident angle of the illumination beam at the air/sample interface should preferably be of the order of 50° to ensure a proper propagation of the illumination beam, so that it can reach the camera 26 and provide an optimized backlight. Indeed, the angle α should be limited to take into account the different interfaces that the illumination beam needs to go through and, hence, limit losses by reflection when going through these different interfaces. The incident angle might also be adjusted as a function of the shape and/or dimensions of the different vessels used during operation. Further, the picking axis can be perpendicular to the vessel support 5 (or to the air/sample interface) or inclined with an angle potentially as great as 90° with respect to the normal direction when the picking axis is horizontal (parallel to the vessel support 5) as illustrated on Fig. 3a and 3b which will be explained below in more detail. Accordingly, all in all, depending also on the nature of the sample liquid or gel, the maximum value for the angle α should be approximately 140° (-50°+90°).

According to a preferred variant embodiment, the illumination device 30 can be connected to the frame 10 of the apparatus 1 in such a manner that it can be driven inside the xy plane with respect to the frame 10.

In order to simplify the construction of the apparatus 1, and to lower the risk of interference between the illumination device 30 and the picking head 14, it can advantageously be provided that these two members are arranged on a common supporting arm 36 which is itself mounted on the frame 10 in such a manner that it can be driven in translation inside the xy plane with respect to the frame 10.

It can then be further preferred, for the sake of simplicity and reliability, that the camera 26 is also mounted on the frame 10 in such a manner that it can be driven in translation inside the xy plane with respect to the frame 10.

In this case, the imaging axis defined by the intersection of the optical path between the illumination device 30 and the camera 26 presents a unique angle with respect to the see-through window 28, whatever the considered picking position. In other words, parallel imaging axes correspond then to the different possible picking positions in a sample. These imaging axes might be either perpendicular to the see-through window 28, or they can be angled with respect to the see-through window 28, without departing from the present invention.

Of course, the one skilled in the art may adapt the present disclosure to implement other methods for driving the illumination device 30 and the camera 26, so that they might both follow more complex movements without going beyond the scope of the present invention as defined in the appended claims. For instance, it is possible to provide that the illumination device 30 and the camera 26 can be driven so as to be tilted with respect to the frame 10 of the sorting apparatus 1.

Generally, it might be advantageous to provide that the image acquisition device comprises a reflective member 38, for instance a mirror or a prism, to change the direction of the propagation of the light along the optical path 32 with respect to the imaging axis, as illustrated in the embodiment of Figs. 2a and 2b. Thanks to this provision, the height of the image acquisition device (along the z axis) can be limited, which can be more particularly useful when the sorting apparatus is used within a standard laminar flow hood, the opening of which has typically a limited height (generally of the order of 20 cm to 30 cm).

In a similar manner, it might be advantageous to provide the image acquisition device with an optical system thanks to which the focal distance can be adjusted, for example at least one focusing lens 40 as illustrated in Figs. 2a and 2b. Thanks to this feature, the focus height in a sample can be adjusted to take into account biological entities which would have picking positions at different heights (in a gel for example) or to take into account the fact that vessels may have different bottom thicknesses.

Figs. 2, 3 and 4 now illustrate different variant embodiments of the present invention.

In a first variant illustrated in Figs. 2a and 2b, the illumination device 30 and the image acquisition device are arranged in such a manner that they can be driven along the xy plane, and such that the optical paths 32 they define between them are always perpendicular to the see-through window 28 (oriented along the z axis here), independently of the picking position.

Accordingly, the pipette tip 16 is inclined with respect to the z axis such that the illumination device 30 and the picking head 14 can be arranged next to each other on the supporting arm 36 while the pipette tip 16 stays out of the optical path 32.

Fig. 2a illustrates how the supporting arm 36 is driven in the xy plane to align the illumination device 30 (and hence the corresponding optical path 32) with a predefined picking position. Once the appropriate position in the xy plane is reached, the picking head 14 can be driven to move along the z axis to place the pipette tip 16 in a proper picking configuration, as illustrated in Fig. 2b. It appears more particularly from this Fig. 2b that the acquisition of images with the required light quality is still possible during the picking operation as far as the pipette tip 16 is kept out of the corresponding optical path 32.

We understand from the illustration of Fig. 2b that the minimum inclination angle α between the optical path 32 (or imaging axis) and the picking axis 34 depends on several parameters among which the dimensions of the biological entities to be picked up and the dimension of the pipette tip opening, as well as the weight and robustness of the biological entities to be picked up which have an impact on the applicable suction force, and thus on the required operating distance between the pipette tip opening and the biological entity to be picked up.

It should be noted that if, in the illustration of Figs. 2a and 2b, it appears that the connecting member (not directly visible) provided on the picking head 14 to secure a pipette tip 16 therein can be standard (for instance a cylindrical surface on which the pipette tip 16 is force fitted, the pipette tip 16 being here specific to exhibit an inclined cone axis with respect to the connecting member of the picking head 14), other embodiments can be implemented without going beyond the scope of the present invention as provided in the appended claims. It could be provided that the connecting member itself is inclined with respect to the z axis, while the pipette tip 16 could be a standard cone, or the pipette tip 16 could also have a two-part shape with a first connecting part intended to cooperate with the connecting member of the picking head 14, and a second part inclined with respect to the first part, to define the picking axis, inclined with respect to the z axis here.

In another variant embodiment illustrated in Figs. 3a and 3b, the illumination device 30 and the image acquisition device are also arranged in such a manner that they can be driven along the xy plane, and such that the optical paths 32 that they define between them are always perpendicular to the see-through window 28 (oriented along the z axis here), independently of the picking position.

However, in this case the geometry of the pipette tip 16 is different from that of the first variant embodiment. Indeed, the pipette tip 16 has a general elongated shape along the z axis, parallel to the direction of the imaging axis, but it has a side opening instead of the standard opening arranged at its free end, on its longitudinal axis. Thanks to these features, the pipette tip 16 can be arranged next to the illumination device 30 without any risk of impinging the corresponding optical path 32.

In this case again, the picking head 14 is preferably arranged so that it can move along the z axis in the direction to the sample, in order to place the pipette tip in a proper picking configuration, as illustrated in Fig. 3b, from which appears the picking axis 34, inclined with respect to the longitudinal axis of the pipette tip 16 and to the imaging axis. More precisely, in this case the picking axis 34 is close to the horizontal direction, i.e. parallel to the bottom of the source vessel 2, and the angle α is approximately of 90°.

In another variant embodiment illustrated in Figs. 4a and 4b, the illumination device 30 and the image acquisition device are also arranged in such a manner that they can be driven along the xy plane, and such that the optical paths 32 they define between them are always inclined with a same angle with respect to the see-through window 28 (oriented along the z axis here), independently of the picking position.

According to this additional variant embodiment, the pipette tip 16 is of conventional elongated shape, oriented along the z axis. Hence, in order to fulfil the requirement of the present invention regarding the inclination between the picking axis and the imaging axis, the illumination device is arranged so that the imaging axis is inclined with respect to the see-through window 28. The camera 26 is appropriately oriented. The reflective member 38 may preferably be further arranged such that its position and/or orientation can be adjusted with respect to the frame 10, to allow a fine adjustment of the imaging axis if necessary.

In this case, when the illumination device 30 and the camera 26 are driven to move from a first picking position to a second picking position, the two optical paths 32, respectively corresponding to the first picking position and to the second picking position, will be parallel to each other (like in the previous variant embodiments), i.e. they will have the same inclination angle with respect to the see-through window 28.

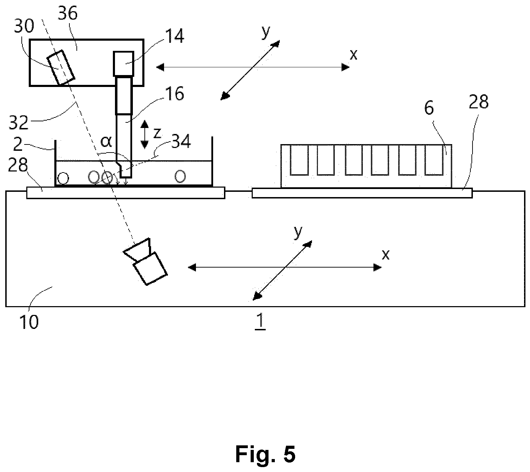

In another variant embodiment illustrated in Fig. 5, features of the embodiments illustrated in Figs. 3a, 3b, 4a and 4b may be combined. According to this additional variant, the illumination device 30 and the image acquisition device are also arranged in such a manner that they can be driven along the xy plane, and such that the optical paths 32 that they define between them are always inclined to the see-through window 28 (oriented along the z axis here), independently of the picking position.

However, in this case, the implementation of inclined optical paths 32 or imaging axes is combined with a side opening pipette tip 16 as described earlier, in connection with Figs. 3a and 3b. Hence, the inclination between pipetting axis and imaging axes exceeds 90° and might increase up to approximately 140° as previously explained.

Generally, the present invention also relates to a method for the selective picking up of at least one biological entity from a source vessel containing a sample including a plurality of biological entities, to be able to preferably deliver this biological entity into a destination vessel. It is understood from what precedes that the method according to the invention should allow a fast, reliable and efficient sorting of biological entities.

On a general basis, the method according to the present invention comprises the steps consisting in:
a) providing a sample including biological entities in a transparent source vessel 2 and place the latter on a vessel support 5 of a sorting apparatus 1 as previously disclosed,
b) controlling the illumination device 30 and the image acquisition device 26 to move them, while keeping the vessel support 5 immobile, so as to define an optical path 32 including a predefined biological entity to be picked up, defining a picking position, and intersecting the see-through window 28 with a corresponding imaging axis,
c) acquiring at least one image of the predefined biological entity to be picked up, and
d) controlling the picking head 14, on which an elongated picking tip 16 is secured, so as to place the elongated picking tip 16 in a picking configuration in which it can pick up the predefined biological entity from the source vessel 2, with its picking axis 34 intersecting the imaging axis substantially at the picking position, the elongated picking tip 16 being kept out of the optical path 32 between the illumination device 30 and the image acquisition device 26,
e) selectively picking up the predefined biological entity, and acquiring at least one image of the predefined biological entity while it is moving between the picking position in the sample and the elongated picking tip 16.

According to another preferred embodiment, when the sorting apparatus 1 comprises a delivery station 8 according to the above-mentioned features, the method according to the invention may advantageously comprise additional steps consisting in:
f) controlling the illumination device 30 and the image acquisition device 26 to move them so as to define an optical path 32 including a predefined delivery position, and intersecting the see-through window 28 of the destination vessel support 7 with a corresponding imaging axis,
g) controlling the picking head 14 so that it can place the elongated picking tip 16 in a delivery configuration corresponding to the predefined delivery position, the elongated picking tip 16 being kept out of the optical path 32 between the illumination device 30 and the image acquisition device 26,
h) delivering the predefined biological entity in the delivery position, and acquiring at least one image of the predefined biological entity while it is moving between the elongated picking tip 16 and the delivery position.

Thanks to the above-described features, a sorting apparatus and a sorting method are provided which make it possible to selectively pick up biological entities from a sample with reduced risks of damages, in a fast, precise and reliable manner.

The one skilled in the art will encounter no particular difficulty to adapt the present disclosure as a function of his needs without going beyond the scope of the present invention as defined by the appended set of claims. It is for instance possible to implement a flexible picking tip which could bend once it is urged against the bottom of the vessel, so as to bring its opening closer to the picking position, hence modifying the picking axis when bending, without going beyond the scope of the present invention.

## Claims

1. Sorting apparatus (1), adapted to allow selective picking up of at least one biological entity from a vessel (2) containing a sample including a plurality of biological entities, said sorting apparatus comprising:
- a picking station (4) including a frame (10), on which a vessel support (5) having a see-through window (28) is mounted for bearing at least one vessel (2), as well as a picking head (14) having a connecting member for securing an elongated picking tip (16) intended to define a picking axis, said picking head (14) being mounted on said frame (10) in such a manner that it can be driven to move with respect to said frame (10) to be able to place the elongated picking tip (16) in a picking configuration, in which it can pick up a predefined biological entity from a picking position inside a vessel (2) while the latter is located on said vessel support (5),
- an image acquisition device (26) arranged so as to be able to acquire through said see-through window (28) an image of at least part of biological entities included in a sample contained in a transparent vessel (2) while the latter is located on said vessel support (5), and
- an illumination device (30) arranged so as to be able to illuminate at least partly a sample contained in a vessel (2) while the latter is located on said vessel support (5),
the sorting apparatus (1) being **characterised in that** said image acquisition device (26) and said illumination device (30) are mounted on said frame (10) in such a manner that they can move with respect to said frame (10) to define between them different optical paths (32) intersecting said see-through window (28) and defining corresponding imaging axis, and
**in that** said picking head (14), said illumination device (30) and said image acquisition device (26) are arranged so that, for any picking position in a vessel (2), which faces said see-through window (28) while the vessel (2) is located on said vessel support (5), an elongated picking tip (16) secured to said picking head (14) can be placed in an appropriate picking configuration with its picking axis intersecting said imaging axis substantially at said picking position, such that the elongated picking tip (16) can be kept out of the corresponding optical path (32) between said illumination device (30) and said image acquisition device (26) during a picking up operation.

2. Sorting apparatus (1) according to claim 1, **characterized in that** said picking head (14) and said illumination device (30) are carried by a supporting arm (36) mounted on said frame (10) in such a manner that it can be driven in translation inside a first plane substantially parallel to said vessel support (5).

3. Sorting apparatus (1) according to claim 2, **characterized in that** said image acquisition device (26) can also be driven in translation with respect to said frame (10) inside a second plane substantially parallel to said vessel support (5).

4. Sorting apparatus (1) according to claim 3, **characterized in that** said image acquisition device (26) is mounted on said frame (10) via said supporting arm (36), so that its movements inside said second plane are synchronized with the movements of said illumination device (30) in said first plane.

5. Sorting apparatus (1) according to any of the preceding claims, **characterized in that** said illumination device (30) and said image acquisition device (26) are connected to said frame (10) in such a manner that each of them can be tilted with respect to said frame (10).

6. Sorting apparatus (1) according to any of the preceding claims, **characterized in that** said picking head (14) and said illumination device (30) are arranged so that the picking axis and said imaging axis can intersect at said picking position with an angle α comprised between 5° and 140°.

7. Sorting apparatus (1) according to any of the preceding claims, **characterized in that** said image acquisition device (26) includes a reflective member (38) to change the light propagation direction with respect to said imaging axis.

8. Sorting apparatus (1) according to any of the preceding claims, **characterized in that** said image acquisition device (26) includes an adjustable focusing system (40) adapted to take into account different possible distances between said picking position and said vessel support (5).

9. Sorting apparatus (1) according to any of the preceding claims, **characterized in that** it is arranged in such a manner that said imaging axis can have a same angle with respect to said see-through window (28) for different picking positions.

10. Sorting apparatus (1) according to claim 9, **characterized in that** it is arranged in such a manner that said imaging axis can have a same inclination angle with respect to the normal direction to said see-through window (28) for different picking positions.

11. Sorting apparatus (1) according to any of the preceding claims, **characterized in that** said image acquisition device (26) is adapted for microscopy imaging, preferably for bright field imaging and/or fluorescence imaging.

12. Sorting apparatus (1) according to any of the preceding claims, **characterized**
**in that** it further comprises a delivery station (8) including a destination vessel support (7) having a see-through window (28) for bearing at least one destination vessel (6),
**in that** said picking head (14) can be driven in such a manner that an elongated picking tip (16) secured thereon, intended to define a delivery axis, can be placed in a delivery configuration in which a picked-up predefined biological entity can be delivered in a predefined delivery position inside said destination vessel (6),
**in that** said illumination device (30) and said image acquisition device (26) can be driven to define an optical path (32) through the see-through window (28) of said destination vessel support (7) with a predefined imaging axis, and
**in that** said picking head (14), said illumination device (30) and said image acquisition device (26) are arranged so that, for any delivery position in a destination vessel (6), which faces said see-through window (28) while the destination vessel (6) is located on said destination vessel support (7), an elongated picking tip (16) secured to said picking head (14) can be placed in a corresponding delivery configuration with its delivery axis intersecting said imaging axis substantially at said delivery position, such that the elongated picking tip (16) can be kept out of the corresponding optical path (32) between said illumination device (30) and said image acquisition device (26) during a delivering operation.

13. Method for the selective picking up of at least one biological entity from a source vessel (2) containing a sample including a plurality of biological entities, **characterized in that** it comprises the steps consisting in:
a) providing a sample including biological entities in a transparent source vessel (2) and place the latter on a vessel support (5) of a sorting apparatus (1) according to any of claims 1 to 12,
b) controlling said illumination device (30) and said image acquisition device (26) to move them, while keeping said vessel support (5) immobile, so as to define an optical path (32) including a predefined biological entity to be picked up, defining a picking position, and intersecting said see-through window (28) with a corresponding imaging axis,
c) acquiring at least one image of said predefined biological entity to be picked up, and
d) controlling said picking head (14), on which an elongated picking tip (16) is secured, so as to place said elongated picking tip (16) in a picking configuration in which it can pick up said predefined biological entity from said vessel (2), with its picking axis intersecting said imaging axis substantially at said picking position, said elongated picking tip (16) being kept out of said optical path (32) between said illumination device (30) and said image acquisition device (26),
e) selectively picking up said predefined biological entity, and acquiring at least one image of said predefined biological entity while it is moving between said picking position in said sample and said elongated picking tip (16).

14. Method according to claim 13, **characterized in that** said illumination device (30) and said image acquisition device (26) are controlled to move in a substantially synchronized manner in step b).

15. Method according to claim 13 or 14, **characterized in that** said illumination device (30) and said image acquisition device (26) are controlled to maintain a substantially constant angle between said imaging axis and said see-through window (28) while they are moving in step b).

16. Method according to any of claims 13 to 15, when it is implemented on a sorting apparatus (1) according to claim 12, **characterized in that** it comprises additional steps consisting in:
f) controlling said illumination device (30) and said image acquisition device (26) to move them so as to define an optical path (32) including a predefined delivery position, and intersecting the see-through window (28) of said destination vessel support (7) with a corresponding imaging axis,
g) controlling said picking head (14) so that it can place said elongated picking tip (16) in a delivery configuration corresponding to said predefined delivery position, said elongated picking tip (16) being kept out of said optical path (32) between said illumination device (30) and said image acquisition device (26),
h) delivering said predefined biological entity in said delivery position, and acquiring at least one image of said predefined biological entity while it is moving between said elongated picking tip (16) and said delivery position.
